(19) Europäisches Patentamt
European Patent Office
Office européen des brevets

(11) Veröffentlichungsnummer: **0 488 079 A1**

(12) **EUROPÄISCHE PATENTANMELDUNG**

(21) Anmeldenummer: **91119958.6**

(51) Int. Cl.[5]: **C07D 473/06**, A61K 31/52

(22) Anmeldetag: **22.11.91**

(30) Priorität: **22.11.90 HU 723690**

(43) Veröffentlichungstag der Anmeldung: **03.06.92 Patentblatt 92/23**

(84) Benannte Vertragsstaaten: **AT BE CH DE ES FR GB GR IT LI LU NL SE**

(71) Anmelder: **CHINOIN Gyògyszer és Vegyészeti Termékek Gyára RT.**
**To utca 1-5**
**H-1045 Budapest IV(HU)**

(72) Erfinder: **Korbonits, Dezsö, Dr.**
**27/d, Vérhalom u.**
**H-1025 Budapest(HU)**
Erfinder: **Héja, Gergely, Dr.**
**27, Násznagy u.**
**H-1131 Budapest(HU)**
Erfinder: **Szomor née Wendele, Mária**
**2, Attila u.**
**H-1013 Budapest(HU)**
Erfinder: **Minker, Emil, Dr.**
**39/b, Nemestakacs u.**
**Szeged(HU)**
Erfinder: **Virág, Sándor, Dr.**
**29/a, Sallai I. u.**
**H-1136 Budapest(HU)**
Erfinder: **Debreczeni, L ránd, Dr.**
**7/a, Béla k. u.**
**H-1125 Budapest(HU)**
Erfinder: **Körmöczy, Péter, Dr.**
**33, Uri u.**
**H-1014 Budapest(HU)**
Erfinder: **Szab , Mária**
**29, Amfiteátrum u.**
**H-1031 Budapest(HU)**
Erfinder: **Szatmári, István, Dr.**
**59/a, Ménesi-ut**
**H-1118 Budapest(HU)**
Erfinder: **T th née It , Katalin**
**74, Veres Péter ut**
**H-1163 Budapest(HU)**
Erfinder: **Mármarosi née Kellner, Katalin**
**19, Ybl M. st.**
**H-2051 Biatorbágy(HU)**

(74) Vertreter: **Patentanwälte Beetz - Timpe - Siegfried - Schmitt-Fumian- Mayr**
**Steinsdorfstrasse 10**
**W-8000 München 22(DE)**

(54) **Oxadiazolylmethylpurine, ihre Herstellung und ihre pharmazeutische Verwendung.**

(57) Die Erfindung betrifft neue 3H-Purine der allgemeinen Formel I

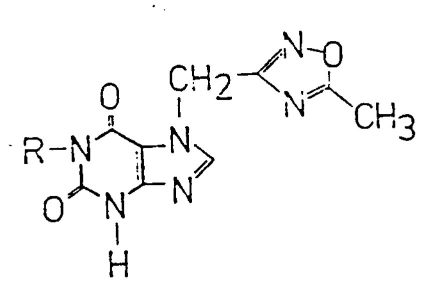

(I),

in der R H oder $C_{1-4}$-Alkyl bedeutet,
und
ihre physiologisch verträglichen Salze sowie ihre Herstellung und Arzneimittel, welche die Verbindungen der

EP 0 488 079 A1

allgemeinen Formel I und ihre Salze enthalten.
Die Verbindungen der Formel I stellen hochwirksame Broncholytika und Antiasthmatika dar, deren besonderer Vorteil darin besteht, daß sie keine unerwünschten Nebenwirkungen auf das Zentralnervernsystem und keine unerwünschten Herzwirkungen aufweisen.

Die Erfindung betrifft Oxadiazolylmethyl-3H-purine der allgemeinen Formel I,

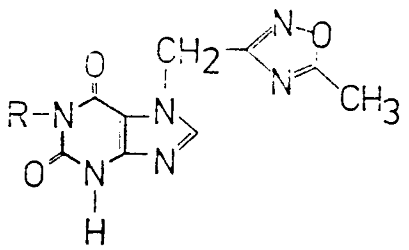

(I),

in der R Wasserstoff oder geradkettiges oder verzweigtes $C_{1-4}$-Alkyl bedeutet, und ihre Salze, insbesondere ihre physiologisch verträglichen Salze und Verfahren zu ihrer Herstellung sowie pharmazeutische Präparate, welche die Verbindungen der allgemeinen Formel I bzw. ihre Salze enthalten.

Die Verbindungen der allgemeinen Formel I sind in erster Linie zur Therapie bei Erkrankungen der Luftwege anwendbar.

Bei Erkrankungen der Atemwege werden zur Linderung des Hustens seit langem unterschiedliche Alkaloide und deren Derivate eingesetzt, z.B. von den Verbindungen mit Morphinstruktur das Codein.

Diese Verbindungen haben jedoch zahlreiche, vom Zentralnervensystem ausgehende unerwünschte Nebenwirkungen. Deswegen gehen in neuerer Zeit die Bestrebungen dahin, Hustenlinderungsmittel mit weniger Nebenwirkungen zu entwickeln.

Zu diesen neueren Hustenlinderungsmitteln gehören verschiedene 3,5-disubstituierte 1,2,4-Oxadiazole, von denen mehrere in der Humanmedizin breit zur Anwendung kommen (Oxolamin, Prenoxdiazin).

Bekannt ist auch, daß bei Erkrankungen der Luftwege wegen ihrer broncholytischen Wirkung bevorzugt verschiedene Xanthinderivate verwendbar sind, von denen das Theophyllin das bekannteste ist.

Es ist ferner eine durch Kombination des 1,2,4-Oxadiazolrings und des Xanthingerüsts entstandene Verbindungsgruppe bekannt, deren Vertreter gleichzeitig eine hustenlindernde und eine broncholytische Wirkung haben (HU-A-190 377 und 197 574).

Bei den therapeutisch eingesetzten Xanthinderivaten ist das Wasserstoffatom der Stickstoffatome N-1, N-7 und N-3 des Purinringes auf unterschiedliche Weise substituiert; allen Verbindungen ist jedoch gemeinsam, daß das Stickstoffatom N-3 immer substituiert ist.

Überraschenderweise wurde nun gefunden, daß die neuen Oxadiazolylmethyl-3H-purine der allgemeinen Formel I sehr vorteilhafte pharmakologische Wirkungen aufweisen und deshalb insbesondere bei Erkrankungen der Atmungsorgane, ganz besonders bevorzugt in Fällen von mit Hustenanfällen einhergehendem Asthma bronchiale, anwendbar sind.

Die hustenlindernde Wirkung der Verbindung Ia als Vertreter der Verbindungen der Formel I

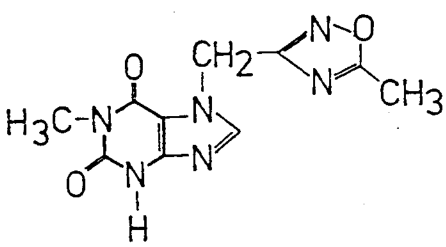

(Ia)

wurde mit der der therapeutisch eingesetzten Wirkstoffe Codein und Dextromethorphan, die broncholytische und antiasthmatische Wirkung mit der des für seine broncholytische Wirkung gut bekannten Theophyllins verglichen.

Die hustenlindernde Wirkung der Verbindung Ia wurde an Katzen untersucht, an denen durch elektrische Reizung des Nervus laryngeus Husten ausgelöst worden war (R. Domenjoz, Arch. Exp. Path. 1952, 19). Die zu untersuchenden Verbindungen wurden i.v. beziehungsweise mit Schlundsonde p.o. verabreicht. Die Experimente zeigten, daß die Verbindung der Formel Ia bei beiden Verabreichungsarten eine bedeutende hustenlindernde Wirkung aufweist. Die Stärke der Wirkung entspricht der der Referenzsubstanzen, die Zeitdauer der Wirkung ist jedoch sowohl bei i.v.- als auch bei p.o.-Verabreichung länger als die der Referenzsubstanzen (Tabelle 1).

Tabelle 1

| Hustenlindernde Wirkung bei an Katzen durch elektrische Reizung hervorgerufenem Husten, $ED_{50}$ ± Standardabweichung bei Verabreichung p.o. | |
|---|---|
| Verbindung | $ED_{50}$ (±S.D.),[mg/kg]p.o. p <0,001 |
| Ia | 3,74 ± 0,2 |
| Codeïn HCl | 4,33 ± 0,1 |
| Dextromethorphan HBr | 3,89 ± 0 2 |

Die hustenlindernde Wirkung wurde auch an Meerschweinchen untersucht, an denen die Dämpfung des durch einen 15 %-igen Citronensäurespray ausgelösten Hustens bestimmt wurde (I. Erdély, L. Tardos, Arzneim. Forsch. 1966, 617). Die hustenlindernden Mittel wurden eine Stunde vor der Messung oral verabreicht. Auch in diesem Test erwies sich die Verbindung Ia als starkes Hustenlinderungsmittel; ihre Wirkung übertraf die der Referenzsubstanzen (Tabelle 2).

Tabelle 2

| Hustenlindernde Wirkung bei an Meerschweinchen durch einen 15 %-igen Citronensäurespray hervorgerufenem Husten, $ED_{50}$ ± Standardabweichung eine Stunde nach p.o. Verabreichung | |
|---|---|
| Verbindung | $ED_{50}$ (±S.D.),[mg/kg]p.o. p <0,001 |
| Ia | 3,04 ± 1,10 |
| Codeïn HCl | 12,84 ± 4,40 |
| Dextromethorphan HBr | 5,26 ± 1,72 |

Die Verbindung Ia hat im Gegensatz zu den Referenzsubstanzen keine atmungsdämpfende Wirkung, sondern schützt sogar sowohl in In-vitro- als auch bei In-vivo-Experimenten vor den Wirkungen der Bronchoconstrictoren. Am glatten Luftröhrenmuskel des Meerschweinchens ist die Wirkung der Verbindung Ia der des Theophyllins ähnlich, wenn die Wirkung gegen Histamin in einer kumulativen Dosisreihe untersucht wird (Tabelle 3).

Tabelle 3

| Hemmung der constrictorischen Wirkung des Histamins am isolierten glatten Luftröhrenmuskel des Meerschweinchens | | |
|---|---|---|
| Verbindung | p d 2 | $E_{max}$(%) |
| Ia | 5,48 ± 1,42 | 36 |
| Theophyllin | 5,38 ± 1,24 | 44 |

Die Hemmung des durch Histamin ausgelösten Anstiegs des Widerstands der Luftwege wurde an Katzen untersucht (M.A. Wassermann, D.W. Du Charme, Prostaglandins, 1977, 255).

Die Hemmung des durch i.v.-Gabe von 3 µg/kg Histamin ausgelösten Anstiegs des Widerstands der Luftwege wurde für die Verbindung Ia und für Theophyllin nach introduodenaler Verabreichung von 4 mg/kg Wirkstoff über das Zeitintervall von der 10. bis zur 120. Minute gemessen. Die Wirkung der Verbindung Ia erwies sich als fast doppelt so stark wie die das Theophyllins und hielt längere Zeit an (Tabelle 4).

Tabelle 4

| Senkung des durch Histamin ausgelösten Anstiegs des Widerstands der Luftwege nach 4 mg/kg Wirkstoff, intraduodenal | | |
|---|---|---|
| Verbindung | Wirkungsmaximum | rel.Wirkungsstärke |
| Ia | 28,2 | 1,79 |
| Theophyllin | 15,7 | 1,00 |

Die Verbindung Ia zeigte eine bedeutende und günstige Wirkung gegen an Meerschweinchen experimentell ausgelöstes Asthma (Anaphylaxe).

Die Meerschweinchen wurden durch eine einmalige Gabe von 1 µg/kg Ovalbumin i.v. sensibilisiert (Andersson, Brattsand, Br. J. Pharmacol. 1982, 139) und bekamen nach 40 Tagen die zu untersuchenden Verbindungen in einer Dosis von 10 mg/kg i.v. Die narkotisierten, muskelrelaxierten und künstlich beatmeten Tiere erhielten 3 mg/kg Ovalbumin intratracheal, und zu unterschiedlichen Zeitpunkten wurde gemessen, wie stark die zu untersuchenden Verbindungen den Anstieg des Widerstandes der Luftwege zu kompensieren vermochten. Die Ergebnisse sind in der Tabelle 5 zusammengestellt.

Versuchsprotokoll

Gruppen: Kontrolle, Verbindung Ia, Theophyllin (jeweils 6 Tiere). Nach Narkose Präparation und Verabreichung eines Muskelrelaxans Beatmung

1. Messung: 5 min nach Injizierung von 10 mg/kg Wirkstoff, dann intratracheale Provokation (Minute 0)
2. Messung: 3 min nach intratrachealer Provokation
3. Messung: 6 min nach intratrachealer Provokation
4. Messung: 12 min nach intratrachealer Provokation
5. Messung: 24 min nach intratrachealer Provokation.

Die Beatmung erfolgte mit positivem Druck bei einer Frequenz von 90/min und einem Volumen von 3 ml.

Die Kontrollgruppe erhielt physiologische Kochsalzlösung. Der Wert $\delta$ ist die Abweichung von dem eigenen, nach der Verabreichung der Dosis von 10 mg/kg Wirkstoff gemessenen Wert (Eigenkontrolle).

Tabelle 5

Änderung des Beatmungswiderstands (R = Pa/ml/s) an sensibilisierten Meerschweinchen nach intratracheal verabreichtem Ovalbumin

| | Gruppe | | |
|---|---|---|---|
| | Kontrolle | Ia | Theophyllin |
| Körpermasse, (g ) | 635±40 | 731±36 | 716±18 |
| Zeitpunkt | Widerstandswerte und ihre Änderung | | |
| nach 10 mg i.v. | 10,72±1,50 | 8,07±0,93 | 11,04±0,26 |
| nach 3 Minuten | 6,53±3,28 | 9,21±0,84 | 12,87±1.06 |
| δ: | -4,20±3,74 | 1,14±0,77 | 1,83±1.05 |
| nach 6 Minuten | 11,81±3,74 | 9,41±0,91 | 14,65±0,93 |
| δ: | 1,09±1,80 | 1,34±0,96 | 3,61±1,04 |
| nach 12 Minuten | 16,32±2,15 | 10,10±0,94 | 14,18±0,54 |
| δ: | 5,60±1,02 | 2,03±0,44 | 3,14±0,65 |
| nach 24 Minuten | 18,16±2,45 | 9,92±1.11 | 14,11±0,51 |
| δ: | 7,44±1,55 | 1,85±0,64 | 3,07±0,39 |

Aus der Tabelle 5 ist ersichtlich, daß die Verbindung Ia das experimentelle Asthma an Meerschweinchen bedeutend zu senken vermag, was in der Verringerung des Widetstandes (R) der Luftwege zum Ausdruck kommt. Die Wirkung ist stärker als die der Kontrollsubstanz Theophyllin.

Die broncholytische und antiasthmatische Wirkung der Verbindung Ia ist auch deshalb bemerkenswert, weil die Verbindung die für Theophyllin charakteristischen unerwünschten Nebenwirkungen auf das Zentralnervensystem und das Herz nicht aufweist.

Die Verbindung Ia hat darüber hinaus außerordentlich günstige Toxizitätswerte. Der an Mäusen bei p.o.-Verabreichung gemessene $DL_{50}$-Wert liegt über 2000 mg/kg. Unter Berücksichtigung der Toxizitätswerte der als Referenz verwendeten Wirkstoffe Codein, Dextromethorphan und Theophyllin ist der therapeutische Index der Verbindung Ia um mehr als eine Größenordnung besser als der der Referenzsubstanzen (Codein.HCl $DL_{50}$ an Mäusen: 359,2 mg/kg p.o.; Dextromethorphan.HBr $DL_{50}$ an Mäusen: 165,9 mg/kg p.o.; Theophyllin, $LD_{50}$ an Mäusen: 215 mg/kg p.o.). Ein besonderer Vorteil der Verbindung Ia liegt darin, daß sie trotz ihrer mäßigen Wasserlöslichkeit bei oraler Verabreichung auch ohne Salzbildung vom Organismus ausgezeichnet und schnell resorbiert wird.

Die erfindungsgemäßen Verbindungen werden vorteilhaft hergestellt, indem man eine Verbindung der allgemeinen Formel II,

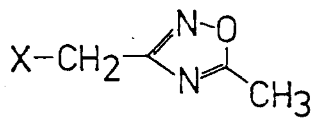

(II),

in der X für Halogen oder Sulfonsäureester steht, mit einem Xanthin der allgemeinen Formel III,

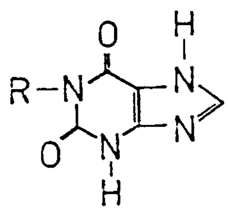

(III),

worin die Bedeutung von R die gleiche wie oben ist, in einem organischen Lösungs- und/oder Verdünnungsmittel, vorzugsweise Dimethylformamid, oder einem Alkohol, vorzugsweise Butanol, in Gegenwart einer anorganischen Base, vorzugsweise einem Alkalihydroxid oder Alkalicarbonat, insbesondere Kaliumcarbonat, umsetzt, oder ein Salz, insbesondere ein Alkalisalz, vorzugsweise das Natrium- oder Kaliumsalz, des Xanthins der allgemeinen Formel III in einem organischen Lösungs- und/oder Verdünnungsmittel mit der Verbindung der allgemeinen Formel II bei 20-150 °C, vorzugsweise bei 40-100 °C, umsetzt.

Die Verbindungen der allgemeinen Formel I haben einen schwach sauren Charakter. Ihre Salze mit Alkalimetallen werden vorzugsweise gebildet, indem man ein äquimolares Gemisch aus der Verbindung der allgemeinen Formel I und einem Alkalihydroxid, insbesondere Natriumhydroxid oder Kaliumhydroxid, in Wasser auflöst und dann eindampft. Die erhaltenen Natrium- bzw. Kaliumsalze sind in Wasser löslich.

Die Verbindungen der allgemeinen Formel I und ihre Salze können als solche oder in Form von Arzneimittelformulierungen in der Medizin angewendet werden. Die Arzneimittelformulierungen können z.B. Sirupe, Tabletten, Dragées oder Kapseln sein und die den Wirkstoff zusammen mit üblichen Hilfs- und/oder Trägerstoffen, z.B. Streckmitteln und sonstigen Hilfsmitteln, enthalten. Die Formulierungen können 0,2 bis 100% Wirkstoff enthalten. Die tägliche Dosis liegt, abhängig von Alter, Körpergewicht und Zustand des Kranken sowie von der Art der Verabreichung und der angestrebten Wirkung, bei etwa 1-500 mg.

Die Erfindung wird im folgenden anhand von Ausführungsbeispielen näher erläutert.

Beispiel 1

3,32 g 1-Methylxanthin (K.A. Chkhidvadze, O. Magidson, Med. Prom. SSSR, 1958, 16) und 1,45 g Kaliumcarbonat werden in 40 ml Dimethylformamid suspendiert. Das Gemisch wird bei 85 °C 20 min lang gerührt und dann bei der gleichen Temperatur innerhalb von 15 min tropfenweise mit 3,43 g 3-Chlormethyl-5-methyl-1,2,4-oxadiazol (J. Wood, DE 19 15 495) versetzt. Das Gemisch wird noch 90 min lang bei der angegebenen Temperatur gerührt und dann heiß filtriert; anschließend wird das Filtrat unter vermindertem Druck zum Trocknen eingedampft. Der Rückstand wird mit 15 ml Wasser verrieben; das feste Produkt wird dann abgesaugt und mit Wasser gewaschen. Das Rohprodukt (4,3 g) wird zuerst aus 38 ml 10 %-iger Salzsäure und dann unter Klären mit Aktivkohle aus 50 %-igem wäßrigen Ethanol umkristallisiert. Auf diese Weise erhält man 3,0 g (60 %) 1-Methyl-7-(5-methyl-1,2,4-oxadiazol-3-yl)-methyl-3,7-dihydro-3H-purin-2,6-dion (Verbindung Ia). F. 243-245 °C.

**$^1$H NMR ( $D_6$ DMSO): $\delta$ = 2,56 (s, 3H, $CH_3$); 3,13 (s, 3H, $CH_3$); 5,65 (s, 2H, $CH_2$); 8,1 (s, =CH-); 12,16 (vb, 1H, NH).**

Beispiel 2

Unter Schütteln werden 3,5 g 1-Methylxanthin in 8,14 ml 10 %-iger Natronlauge gelöst; aus der Lösung scheidet sich schnell eine Festsubstanz aus. Das Wasser wird dann unter vermindertem Druck abdestilliert, letzte Spuren von Wasser werden aus dem Rückstand durch Azeotropdestillation mit Toluol entfernt. Das auf diese Weise erhaltene 1-Methylxanthin-natrium wird, wie in Beispiel 1 beschrieben, in Dimethylformamid suspendiert und mit 3-Chlormethyl-5-methyl-1,2,4-oxadiazol umgesetzt. Die Verbindung der Formel Ia fällt in der gleichen Ausbeute an.

Beispiel 3

| Herstellung von Tabletten | |
|---|---|
| Verbindung Ia | 2,0 g |
| Stärke | 97,0 g |
| Calciumphosphat | 100,0 g |
| Magnesiumstearat | 1,0 g |
| insgesamt | 200,0 g |

Aus dem pulverisierten Gemisch werden in bekannter Weise 1000 Tabletten zu je 200 mg hergestellt. Jede Tablette enthält 2 mg Wirkstoff.

Beispiel 4

| Herstellung von Sirup | |
|---|---|
| Verbindung Ia | 4,0 g |
| Zitronensirup | 200,0 ml |
| Benzoesäurelösung | 20,0 ml |
| Wasser | 100,0 ml |
| Zuckersirup ad | 1000,0 ml |

Der Wirkstoff wird heiß in Wasser gelöst. Zu der Lösung werden 500 ml Zuckersirup und dann die restlichen Komponenten zugegeben. Schließlich wird mit weiterem Zuckersirup auf ein Endvolumen von 1000 ml aufgefüllt. Der Sirup enthält 4 mg Wirkstoff pro Milliliter.

**Patentansprüche**

**1.** Verbindungen der allgemeinen Formel I,

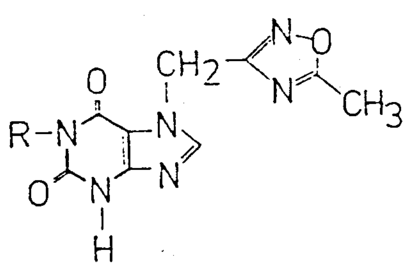

(I),

in der R Wasserstoff oder geradkettiges oder verzweigtes $C_{1-4}$-Alkyl bedeutet, und ihre Salze.

**2.** 1-Methyl-7-(5-methyl-1,2,4-oxadiazol-3-yl)-methyl-3,7-dihydro-3H-purin-2,6-dion und seine Salze.

**3.** Verfahren zur Herstellung der Verbindungen der Formel I nach den Ansprüchen 1 und 2, gekennzeichnet durch

(A) Umsetzung einer Verbindung der allgemeinen Formel II,

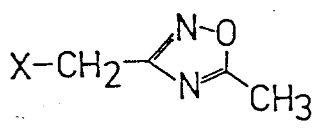

(II),

in der X ein Halogenatom oder eine Sulfonsäureestergruppe bedeutet, mit einer Verbindung der allgemeinen Formel III,

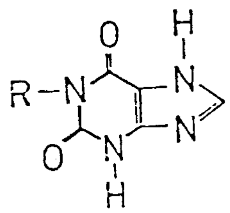

(III),

in der R Wasserstoff oder geradkettiges oder verzweigtes $C_{1-4}$-Alkyl bedeutet,
oder
(B) Umsetzung eines Salzes einer Verbindung der allgemeinen Formel III wie oben definiert mit einer Verbindung der allgemeinen Formel II wie oben definiert.

**4.** Verfahren nach Anspruch 3, dadurch gekennzeichnet, daß die Umsetzung (A) in Gegenwart einer Base, vorzugsweise einer anorganischen Base, insbesondere eines Alkalihydroxids oder Alkalicarbonats, vorgenommen wird.

**5.** Verfahren nach den Ansprüchen 3 und 4, dadurch gekennzeichnet, daß die Umsetzung (A) oder (B) in einem organischen Lösungsmittel und/oder Verdünnungsmittel durchgeführt wird.

**6.** Verfahren nach den Ansprüchen 3 bis 5, dadurch gekennzeichnet, daß die Umsetzung (A) bzw. (B) unter Verwendung von Dimethylformamid und/oder eines Alkohols, vorzugsweise Butanol, durchgeführt wird.

**7.** Verfahren nach den Ansprüchen 3 bis 6, dadurch gekennzeichnet, daß die Umsetzung (A) oder (B) bei einer Temperatur von 20 bis 150 °C und vorzugsweise von 40 bis 100 °C durchgeführt wird.

**8.** Pharmazeutische Zusammensetzungen, gekennzeichnet durch eine oder mehrere Verbindungen der Formel I nach den Ansprüchen 1 und 2 bzw. entsprechender Salze als Wirkstoffe neben üblichen Hilfs- und/oder Trägerstoffen.

**9.** Pharmazeutische Zusammensetzungen nach Anspruch 8, dadurch gekennzeichnet, daß sie 1-Methyl-7-(5-methyl-1,2,4-oxadiazol-3-yl)-methyl-3,7-dihydro-3H-purin-2,6-dion bzw. dessen Salze als Wirkstoffe enthalten.

**10.** Verfahren zur Herstellung der pharmazeutischen Zusammensetzungen nach den Ansprüchen 8 und 9, dadurch gekennzeichnet, daß man eine oder mehrere Verbindungen der Formel I gemäß Anspruch 1 bzw. entsprechende Salze mit physiologisch verträglichen Hilfs- und/oder Trägerstoffen zu Arzneimitteln formuliert.

Europäisches Patentamt

# EUROPÄISCHER RECHERCHENBERICHT

Nummer der Anmeldung

EP 91 11 9958
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| --- | --- | --- | --- |
| Y | EP-A-0 264 081 (CHINOIN GYOGYSZER ES VEGYESZETI TERMEKEK GYARA RT)<br>* Zusammenfassung; Ansprüche; Beispiel 35 * | 1,3,8,10 | C07D473/06<br>A61K31/52 |
| Y | EP-A-0 089 028 (CHINOIN GYOGYSZER ES VEGYESZETI TERMEKEK GYARA RT)<br>* Seite 8, Zeilen 13-14; Seite 9, Zeilen 24-33; Zusammenfassung; Ansprüche 1,2,4,7 * | 1,3,8,10 | |
| A | EP-A-0 315 401 (CHINOIN GYOGYSZER ES VEGYESZETI TERMEKEK GYARA RT)<br>* das ganze Dokument * | 1,3,8,10 | |
| A | EP-A-0 272 596 (ISTITUTO BIOLOGICO CHEMIOTERAPICO "ABC" S.P.A.)<br>* Ansprüche 1,7,8 * | 1,8 | |
| A | EP-A-0 171 005 (BOEHRINGER BIOCHEMIA ROBIN S.P.A.)<br>* Ansprüche 1,8 * | 1,8 | **RECHERCHIERTE SACHGEBIETE (Int. Cl.5 )** |
| A | CHEMICAL ABSTRACTS, vol. 97, no. 7, 16. August 1982, Columbus, Ohio, US;<br>abstract no. 49325B,<br>C.G.A. PERSSON ET AL.: 'Differentiation between bronchodilation and universal adenosine antagonism among xanthine derivatives'<br>Seite 24 ;<br>& Life Sci. 1982, 30(25), 2181-9 | 1,8 | C07D |
| A,D | ARZNEIMITTEL-FORSCHUNG<br>1966, AULENDORF, WÜRTT., DE<br>Seiten 617 - 621;<br>L. TARDOS ET AL.: 'Pharmakologische Untersuchung des neuen Antitussivums 3-(2,2-Diphenyläthyl)-5-(2-piperidinoäthyl)-1,2,4-oxadiazol' | 1,8 | |

-/--

Der vorliegende Recherchenbericht wurde für alle Patentansprüche erstellt

| Recherchenort | Abschlußdatum der Recherche | Prüfer |
| --- | --- | --- |
| BERLIN | 26 FEBRUAR 1992 | HASS C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 91 11 9958
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | MEDICINSKAJA PROMYSLENNOST' SSSR<br>1958, MOSKAU<br>Seiten 16 - 22;<br>K.A. CHKHIDVADSE ET AL.: 'Synthesis of Theophylline from 1-methyl-4-aminouracil'<br>* Seite 18, Schema III, Endprodukt *<br>----- | 1 | |

RECHERCHIERTE SACHGEBIETE (Int. Cl.5 )

Der vorliegende Recherchenbericht wurde für alle Patentansprüche erstellt

| Recherchenort | Abschlußdatum der Recherche | Prüfer |
|---|---|---|
| BERLIN | 26 FEBRUAR 1992 | HASS C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)